(19) Europäisches Patentamt / European Patent Office / Office européen des brevets

(11) **EP 3 315 972 A1**

(12) **EUROPEAN PATENT APPLICATION**

(43) Date of publication:
02.05.2018 Bulletin 2018/18

(51) Int Cl.:
*G01N 33/86* (2006.01)     *G01N 33/49* (2006.01)
*G01N 21/82* (2006.01)

(21) Application number: 17198863.7

(22) Date of filing: 27.10.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 28.10.2016 JP 2016212148

(71) Applicant: SYSMEX CORPORATION
Kobe-shi
Hyogo 651-0073 (JP)

(72) Inventors:
• **Thomas, Merin**
  **Hyogo, 651-0073 (JP)**
• **Yamaguchi, Keiichi**
  **Hyogo, 651-0073 (JP)**
• **Kurono, Hiroshi**
  **Hyogo, 651-0073 (JP)**

(74) Representative: **Hoffmann Eitle**
**Patent- und Rechtsanwälte PartmbB**
**Arabellastraße 30**
**81925 München (DE)**

(54) **BLOOD COAGULATION ANALYSIS METHOD, BLOOD COAGULATION ANALYZER, AND COMPUTER PROGRAM**

(57) A blood coagulation analysis method according to an embodiment includes: calculating blood coagulation time based on data which represents a coagulation curve indicating a temporal change in an optical detection value of a blood specimen added with a measurement reagent; and determining an early reaction error by using a result of determination of conformity between a shape of the coagulation curve represented by the data and a shape of a reference coagulation curve.

EP 3 315 972 A1

**Description**

BACKGROUND

The disclosure relates to blood coagulation analysis.

Blood coagulation time is measured in blood coagulation analysis. The blood coagulation time is calculated using a coagulation curve which is obtained through optical detection of a change in turbidity of a blood specimen. There is a case where the coagulation curve shows an early reaction in the early section. The early reaction causes a turbidity change different from that in a normal coagulation reaction, and may take place in blood which is collected from, for example, a subject with a high volume of heparin injected or from a subject with disseminated intravascular coagulation (DIC).

An early reaction error (ERE) is an error in which the early reaction is erroneously recognized as a coagulation reaction and the coagulation time is erroneously calculated. Japanese Patent No. 5889480 (Patent Literature 1) discloses a method of detecting the early reaction error. In Patent Literature 1, the early reaction error is detected by monitoring a reaction status of at least one checkpoint set on a coagulation reaction curve or of a check region set for the coagulation reaction curve.

Patent Literature 1: Japanese Patent No. 5889480.

SUMMARY

The frequency of early reactions is not high because the occurrence probability of early reactions is approximately 1%. The method of Patent Literature 1 is excellent in that the method can detect an early reaction error, without overlooking, by supposing that an early reaction might take place if a coagulation curve has a characteristic of the early reaction.

On the other hand, the inventors have found that an early reaction error does not always occur simply because an early reaction takes place. Even when the early reaction takes place in a coagulation curve, for example, there is a case where a coagulation time is correctly calculated and the early reaction error does not occur. To be more specific, even when the early reaction takes place, if a coagulation reaction normally proceeds after that, the coagulation curve on the whole may have almost the same shape as that of a normal coagulation curve as a result. The inventors have found that in such a case, the coagulation time is expected to be calculated correctly even when the early reaction takes place, and hence the situation does not have to be dealt with as the early reaction error.

A first aspect of the disclosure is a blood coagulation analysis method. In one or more embodiments, the method includes calculating blood coagulation time using data which represents a coagulation curve indicating a temporal change in an optical detection value of a blood specimen added with a measurement reagent. The optical detection value of the blood specimen is a value representing an optical characteristic such as the absorbance of the blood specimen and is detected based on light emitted onto the blood specimen. The optical detection value is, for example, a detection value of the amount of light which has transmitted through the blood specimen or of the amount of light which has scattered from the blood specimen. The amount of light detected changes due to, for example, the change in absorbance or turbidity of the blood specimen.

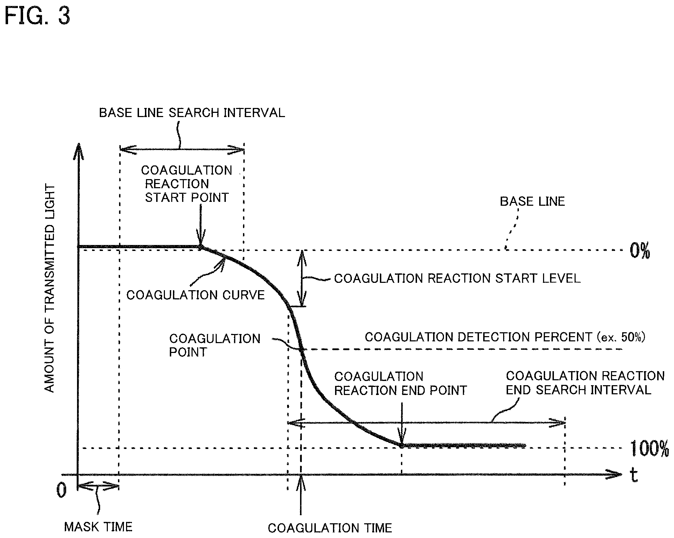

The method includes determining an early reaction error by using a result of determination of conformity between a shape of the coagulation curve represented by the data and a shape of a reference coagulation curve. The reference coagulation curve is a coagulation curve as a reference for the conformity determination. The reference coagulation curve may be set for the general coagulation curve without an error as illustrated in Fig. 3, for example. The reference coagulation curve at least includes a portion of the coagulation curve within the section from the coagulation reaction start point to the coagulation reaction end point so that the reference coagulation curve can have the shape of a general coagulation curve.

If an early reaction takes place, the shape of the coagulation curve as a whole may become greatly different from the shape of the general coagulation curve. The reliability of the coagulation time calculated from such a coagulation curve is low. However, the coagulation time calculated from the coagulation curve is reliable if the shape of the coagulation curve as a whole is along the shape of the general coagulation curve even in the presence of the early reaction. Thus, it is useful in the determination of the early reaction error if a curve with a general shape as a coagulation curve is prepared and conformity between the coagulation curve and the reference coagulation curve is considered. In the conformity determination, the coagulation curve does not have to be one represented by data during the entire detection period but may at least include data during a period used to calculate the blood coagulation time. The determination of the early reaction error may be performed by use of information other than the conformity determination result.

The order of the calculation of the blood coagulation time and the determination of the early reaction error is arbitrary and is not particularly limited.

In one or more embodiments, the method may include: calculating blood coagulation time using data which represents a coagulation curve indicating a temporal change in an optical detection value of a blood specimen added with a measurement reagent; determining conformity between a shape of the coagulation curve represented by the data and a shape of a reference coagulation curve; and outputting the blood coagulation time in an output mode depending on a result of the determination of the conformity. One example of the output mode may indicate whether or not the blood coagulation time depending on the conformity determination result is outputted. Another example of the output mode may indicate whether or not a flag depending on the conformity determination result is outputted when the blood coagulation time is outputted. The order of the calculation of the coagulation time and the conformity determination is arbitrary and is not particularly limited.

Another aspect of the disclosure is a blood coagulation analyzer. The analyzer includes a processing unit. In one or more embodiments, the processing unit executes a process of calculating blood coagulation time using data which represents a coagulation curve indicating a temporal change in an optical detection value of a blood specimen added with a measurement reagent. The processing unit executes a process of determining an early reaction error by using a result of determination of conformity between a shape of the coagulation curve data represented by the data and a shape of a reference coagulation curve.

In one or more embodiments, the processing unit is configured to execute: calculating blood coagulation time using data which represents a coagulation curve indicating a temporal change in an optical detection value of a blood specimen added with a measurement reagent; determining conformity between a shape of the coagulation curve represented by the data and a shape of a reference coagulation curve; and outputting the blood coagulation time in an output mode depending on a result of the determination of the conformity.

Another aspect of the disclosure is a computer program which causes processor of a computer to execute processing for blood coagulation analysis. The computer program is stored in computer-readable storage media. In one or more embodiments, the processing for blood coagulation analysis may include calculating blood coagulation time using data which represents a coagulation curve indicating a temporal change in an optical detection value of a blood specimen added with a measurement reagent. The processing for blood coagulation analysis may include determining an early reaction error by using a result of determination of conformity between a shape of the coagulation curve represented by the data and a shape of a reference coagulation curve.

In one or more embodiments, the processing for blood coagulation analysis includes: calculating blood coagulation time using data which represents a coagulation curve indicating a temporal change in an optical detection value of a blood specimen added with a measurement reagent; determining conformity between a shape of the coagulation curve represented by the data and a shape of a reference coagulation curve; and outputting the blood coagulation time in an output mode depending on a determination result for the conformity.

BRIEF DESCRIPTION OF DRAWINGS

Fig. 1 is a flowchart of blood coagulation analysis processing;
Fig. 2 is a block diagram of a blood coagulation analyzer;
Fig. 3 is an explanatory diagram of a percent detection method;
Fig. 4 is an explanatory diagram of a slow reaction check;
Fig. 5 is an explanatory diagram of a start angle check;
Fig. 6 is an explanatory diagram of an early percent check;
Fig. 7 is a flowchart of conformity determination processing;
Fig. 8A is an explanatory diagram of a parameter $c_1$;
Fig. 8B is an explanatory diagram of the parameter $c_1$;
Fig. 9A is an explanatory diagram of a parameter a;
Fig. 9B is an explanatory diagram of the parameter a;
Fig. 10A is a diagram illustrating an output screen;
Fig. 10B is a diagram illustrating an output screen;
Fig. 11A is a diagram illustrating an example of removing an ERE flag;
Fig. 11B is a diagram illustrating an example of removing the ERE flag;
Fig. 12A is a diagram illustrating a first example of retaining the ERE flag;
Fig. 12B is a diagram illustrating the first example of retaining the ERE flag;
Fig. 13A is a diagram illustrating a second example of retaining the ERE flag;
Fig. 13B is a diagram illustrating the second example of retaining the ERE flag;
Fig. 14A is a diagram illustrating a third example of retaining the ERE flag;
Fig. 14B is a diagram illustrating the third example of retaining the ERE flag;
Fig. 15A is a diagram illustrating an example of a slow reaction where the ERE flag is retained;

Fig. 15B is a diagram illustrating an example of the slow reaction where the ERE flag is removed;
Fig. 16A is a diagram illustrating an example of a start angle 1 where the ERE flag is retained;
Fig. 16B is a diagram illustrating an example of the start angle 1 where the ERE flag is removed;
Fig. 17A is a diagram illustrating an example of a start angle 2 where the ERE flag is retained;
Fig. 17B is a diagram illustrating an example of the start angle 2 where the ERE flag is removed;
Fig. 18A is a diagram illustrating an example of an early percent where the ERE flag is retained; and
Fig. 18B is a diagram illustrating an example of the early percent where the ERE flag is removed.

DETAILED DESCRIPTION

[1. Blood Coagulation Analysis]

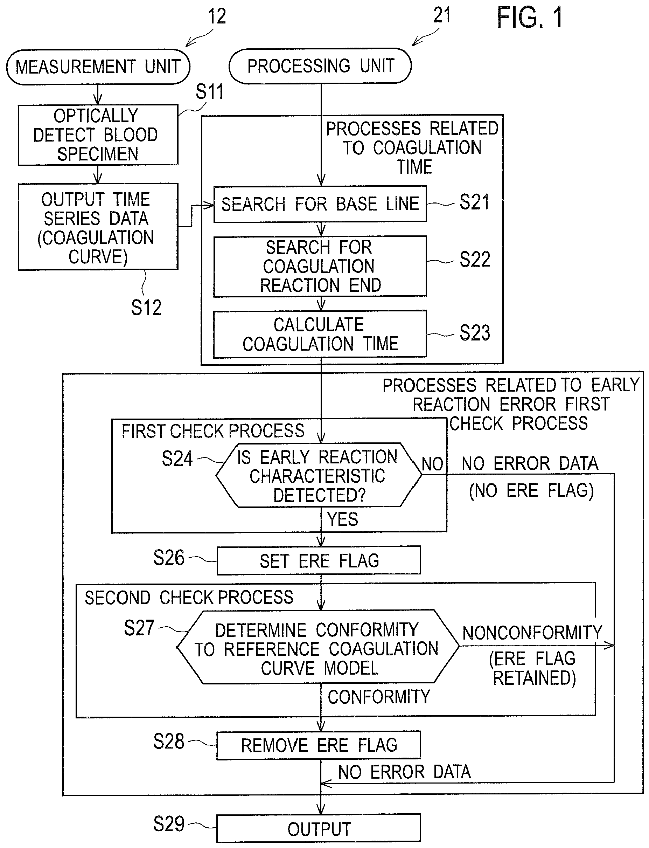

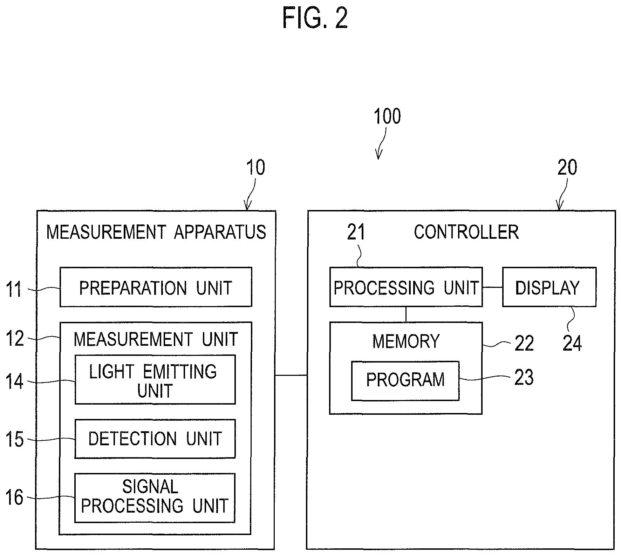

A flowchart illustrated in Fig. 1 indicates blood coagulation analysis processing which includes calculation of blood coagulation time (hereinafter simply referred to as a "coagulation time"). The processes in Fig. 1 are executed by a blood coagulation analyzer 100 illustrated in Fig. 2. The analyzer 100 includes a controller 20. The controller 20 includes a computer which has a processing unit 21 or a processor and a memory 22. The controller 20 may include a display 24 which outputs e.g. a result of processing by the processing unit 21. The memory 22 stores a computer program 23 which causes the processing unit 21 to execute some of the processes in Fig. 1 (step S21 to step S29) to be executed by the processing unit 21.

The analyzer 100 includes a measurement apparatus 10 for measurement of a blood specimen. For example, the measurement apparatus 10 performs measurement for blood coagulation analysis by a coagulation method. The coagulation method includes: preparing a blood specimen by heating a measured amount of blood sample for a certain period of time, followed by addition of a measurement reagent; emitting light onto the blood specimen; and detecting the blood coagulation process as a change in optical characteristic of the blood specimen.

The measurement apparatus 10 includes a preparation unit 11 and a measurement unit 12. The preparation unit 11 prepares the blood specimen by heating the blood sample and adding the measurement reagent to the heated blood sample. The measurement reagent is a reagent for measurement of activated partial thromboplastin time (APTT), for example. The blood specimen is transported to the measurement unit 12. At step S11 of Fig. 1, the measurement unit 12 performs optical detection of the blood specimen.

The measurement unit 12 includes a light emitting unit 14 and a detection unit 15 for the purpose of optical detection. The light emitting unit 14 emits light onto the blood specimen. The light emitting unit 14 is, for example, a halogen lamp or an LED. The detection unit 15 outputs, as an optical detection value, an electric signal corresponding to the amount of light received from the measurement specimen. The detection unit 15 may receive light transmitted through or scattered from the blood specimen. In the following description, the detection unit 15 receives transmitted light.

As the coagulation reaction of the blood specimen proceeds, the amount of light transmitting through the blood specimen changes because the turbidity of the measurement specimen increases. The detection unit 15 of the embodiment detects the blood coagulation process as a change in transmitted light. Note that the amount of light received decreases as the coagulation reaction proceeds in the case of receiving transmitted light, and the amount of light received increases as the coagulation reaction proceeds in the case of receiving scattered light.

The measurement unit 12 includes a signal processing unit 16 which has an analog-to-digital converter. At step S12 of Fig. 1, the signal processing unit 16 converts an optical detection value outputted from the detection unit 15 to digital data, and transmits the digital data to the controller 20. The digital data transmitted to the controller 20 is time series data during a detection period from when the detection unit 15 starts detection to when the detection unit 15 finishes detection. This time series data is coagulation curve data obtained by detecting the blood coagulation process as a temporal change in the amount of transmitted light as described above. The coagulation curve data has a sampling time range of 0.1 seconds, for example. The time from the start of detection to the completion of detection is one hour at most, for example.

At step S21 to step 23 of Fig. 1, the processing unit 21 executes the process of calculating the blood coagulation time based on the received coagulation curve data. In the embodiment, the blood coagulation time to be calculated is the activated partial thromboplastin time (APTT). In the measurement of APTT, it is likely that the early reaction takes place. A description is provided later on how to calculate the blood coagulation time.

From step S24 to step S28, the processing unit 21 executes the processes related to the early reaction error. In the embodiment, the processes related to the early reaction error include a first check process at step S24 and a second check process at step S27. The first check process is a process of detecting a characteristic representative of the early reaction from the coagulation curve. The second check process is a process of determining the conformity of the shape of the coagulation curve to the shape of a reference coagulation curve.

In the embodiment, the first check process is a process of evaluating the coagulation curve itself in order to detect the presence of the early reaction. The second check process, on the other hand, is a process of evaluating a regression curve of the coagulation curve in order to determine whether or not the coagulation curve is one which enables correct calculation of coagulation time. In the embodiment, the regression curve is obtained by regression of the coagulation curve along the reference coagulation curve.

The processes related to the early reaction error include a process of operating an early reaction error (ERE) flag at step S26 and step S28. The ERE flag indicates whether or not the early reaction error has occurred, and is operated according to the results of the first check process and the second check process. In the embodiment, the ERE flag is set if at least one early reaction characteristic is detected in the coagulation curve and if it is determined that the coagulation curve and the reference coagulation curve do not conform to each other. The ERE flag is not set if the coagulation curve and the reference coagulation curve conform to each other, even in the case where an early reaction characteristic is detected.

At step S29, the processing unit 21 outputs the results of the earlier processes to the display 24. The processing results include at least one of an output depending on the ERE flag (an error is displayed, for example) and the coagulation time. The output may be transmission of the processing results to another computer. The details of the processes at step S24 to step S29 are described later.

[2. Coagulation Time]

Fig. 3 illustrates a basic shape of the coagulation curve used to calculate the coagulation time. As illustrated in Fig. 3, the shape of the general coagulation curve is a shape along a sigmoid curve, for example. The sigmoid curve has a shape similar to the final form of the Greek letter sigma or the Latin letter s. It suffices that the sigmoid curve here has a shape similar to the final form of sigma or s and includes what is called a logistic curve or a Gompertz curve, for example. In addition, the sigmoid curve here includes a curve with a first shape the vertical axis value of which increases as the horizontal axis value increases, or a curve with a second shape the vertical axis value of which decreases as the horizontal axis value increases. The shape illustrated in Fig. 3 is the second shape. Here, if the optical detection value is the amount of transmitted light, the general coagulation curve has the second shape illustrated in Fig. 3. On the other hand, if the optical detection value is the amount of scattered light, the general coagulation curve has the first shape.

As illustrated in Fig. 3, in the case of the general coagulation curve without an error, the amount of transmitted light before the coagulation reaction start is almost constant. Once the coagulation reaction starts, the amount of transmitted light decreases monotonically. The amount of transmitted light after the coagulation reaction end is almost constant. Note that if an abnormal reaction occurs such as the early reaction, the shape of the coagulation curve may greatly differ from the basic shape illustrated in Fig. 3.

The coagulation time is calculated assuming that the coagulation curve has the shape as in Fig. 3. Fig. 3 illustrates a percent detection method as an example of the method of calculating coagulation time. The percent detection method is a method in which the amount of transmitted light before the coagulation reaction start is set to 0% as a base line, the amount of transmitted light at the end point of the coagulation reaction is set to 100%, and the time is calculated as the coagulation time until which the amount of transmitted light reaches a coagulation detection percent. The coagulation detection percent is set as a value representing a certain ratio to the difference between the amount of transmitted light on the base line and the amount of transmitted light at the end point of the coagulation reaction. The coagulation detection percent is used to search for a coagulation point at which the amount of transmitted light has changed approximately by a certain ratio (coagulation detection percent) from the base line. The coagulation detection percent is set to a value greater than 0 and less than 100. The coagulation detection percent is set to, for example, 50%.

At step S21 of Fig. 1, in the coagulation curve data, the processing unit 21 searches for a point (coagulation reaction start point) at which the amount of transmitted light is maximum within a base line search section of Fig. 3. The base line search section is a predetermined period which is from the start of detection (for example, a period of 60 seconds from the start of detection), and is a period which excludes a predetermined mask time set immediately after the start of detection (zero-second point). The data in the coagulation curve data corresponding to the base line search section is used for base line search. Note that the mask time is about several seconds, for example. The processing unit 21 decides as the base line the amount of transmitted light at the coagulation reaction start point searched for. Note that here, the base line is referred to as a base optical detection value.

At step S22, the processing unit 21 searches for the coagulation reaction end in the coagulation curve data. The coagulation reaction end is searched for within a coagulation reaction end search section which is a period after a time point where the difference between the base line and the amount of transmitted light exceeds a predetermined coagulation reaction start level (see Fig. 3). The data in the coagulation curve data corresponding to the coagulation reaction end section is used to search for the coagulation reaction end. While the coagulation reaction end is being searched for, the coagulation reaction end point is searched for at which the change in transmitted light becomes small due to the coagulation reaction end. Hereinafter, the amount of transmitted light at the coagulation reaction end point is referred to as an end optical detection value.

At step S23, the processing unit 21 decides the coagulation time based on the percent detection method. To be more specific, the processing unit 21 sets the amount of transmitted light on the base line to 0%, sets the amount of transmitted light at the coagulation reaction end point (end optical detection value) to 100%, and decides the time at which the amount of transmitted light reaches the coagulation detection percent as the coagulation time.

[3. Processes Related to Early Reaction Error]

[3.1 Detection of Characteristics Representative of Early Reaction (First Check Process)]

The processing unit 21 performs processing of determining whether or not there is an error in order to ensure the reliability of the coagulation time calculated at step S23. The error includes the early reaction error. Note that the determination of whether or not there is an error may include the determination of whether or not there is an error other than the early reaction error.

As described above, in the embodiment, the processes related to the early reaction error include the first check process at step S24 of Fig. 1. In the first check process, a characteristic representative of the early reaction is detected in the coagulation curve. The processing unit 21 determines whether or not at least one of the three characteristics is detected in the coagulation curve. At step S24, the processing unit 21 checks whether or not there are the following three characteristics as characteristics representative of the early reaction. Note that the characteristics representative of the early reaction may be one or two of the following three, or may include other characteristics.

- slow reaction: in the coagulation curve, the change time (Time 2 - Time 1) from a first optical detection value TL1 to a second optical detection value TL2 is longer than a max time being a reference period.
- start angle: in the coagulation curve, dH2-dH1 being the change in the optical detection value during a predetermined period (from the 1st check time to the 2nd check time) is greater than Delta being a reference value.
- early percent: in the coagulation curve, the time until which a third optical detection value Check Point is reached is shorter than Limit being reference time.

When at least one characteristic is detected, the processing unit 21 tentatively sets the ERE flag at step S26. If no characteristics representative of the early reaction are detected, the processing unit 21 does not set the flag indicating the early reaction error but finishes the processes related to reaction error. To be more specific, the coagulation time calculated at step S23 is dealt with as one without the early reaction error. Note that if the ERE flag is set, the processing unit 21 continues the processing and performs the second check process at step S27 in order to check whether or not to retain the set ERE flag. The second check process is described later.

Hereinafter, each of the three characteristics is described.

[3.1.1 Slow Reaction Check]

The change rate of the optical detection value is usually very small after a reagent is added to plasma and the resultant reaction proceeds and until the beginning of fibrin formation. As the fibrin formation proceeds, however, a sudden optical change occurs during a short time period. For this reason, it is possible to check whether or not there is a characteristic of the early reaction by investigating the reaction rate at a checkpoint provided at the position of a particular change rate between the start of optical change attributed to fibrin formation and the coagulation end level.

The reaction rate may be obtained by calculating the change rate of the optical detection value at the checkpoint per unit time, or may be calculated as the time required to cause an optical change within a certain range having the checkpoint at the middle. A threshold value is set for the reaction rate (the threshold value can be set experimentally or empirically, for example). If the threshold value is not reached, this means that the characteristic for a "reaction rate error" is detected.

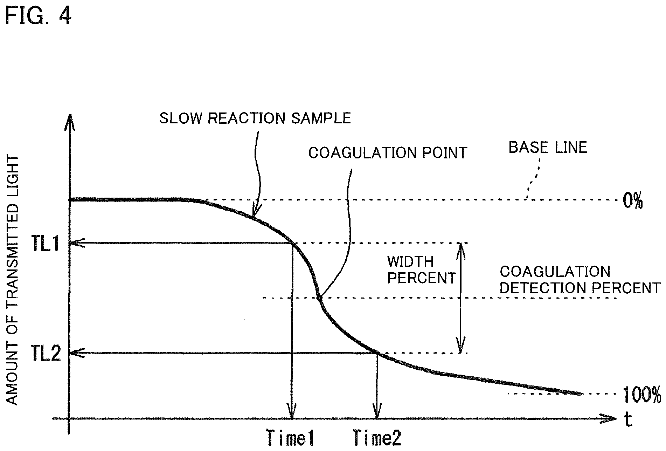

As in Fig. 4, for example, the ERE flag is set if the reaction time (Time 2 - Time 1) in the Width [%] range having the coagulation detection percent in the middle is longer than MaxTime [sec] value. The coagulation detection percent is 50%, for example, the Width [%] is 12%, for example, and MaxTime is 8 [sec], for example. In addition, the ERE flag may be set if the reaction time (Time 2 - Time 1) satisfies the condition that the reaction time is longer than MaxTime [sec] and if there is no point before the coagulation time at which the reaction rate ratio exceeds the reference ratio Ratio.

In comparison with the reaction time taken for the amount of transmitted light of a normal blood sample to change within the Width [%] range, the reaction time is longer taken for the amount of transmitted light of an abnormal blood sample to change within the Width [%] range. Thus, with the threshold value MaxTime set in advance (MaxTime can be determined experimentally or empirically), it is possible to determine the case as the early reaction error where Time 2 - Time 1 > MaxTime is satisfied.

[3.1.2 Start Angle Check]

Since the optical detection amount changes little within the early time range (for example, about 20 seconds for APTT) in the normal coagulation curve, it is possible to judge whether or not there is a characteristic of the early reaction by setting certain two times as checkpoints in the early stage of the coagulation curve, and calculating the change rate of the optical detection value between the checkpoints.

If the change rate of the optical detection amount between the two checkpoints exceeds a threshold value for start angle check set in advance, it is detected that there is a characteristic of the early reaction. Moreover, another threshold value is also set for the change rate of the optical detection amount from the base line to the coagulation reaction end point. If the threshold value is not reached, it is possible to display an error flag as an error in measuring the coagulation time without displaying the coagulation time. In the case of the threshold value or more, it is possible to display the coagulation time after displaying the error flag to indicate a measurement error has occurred.

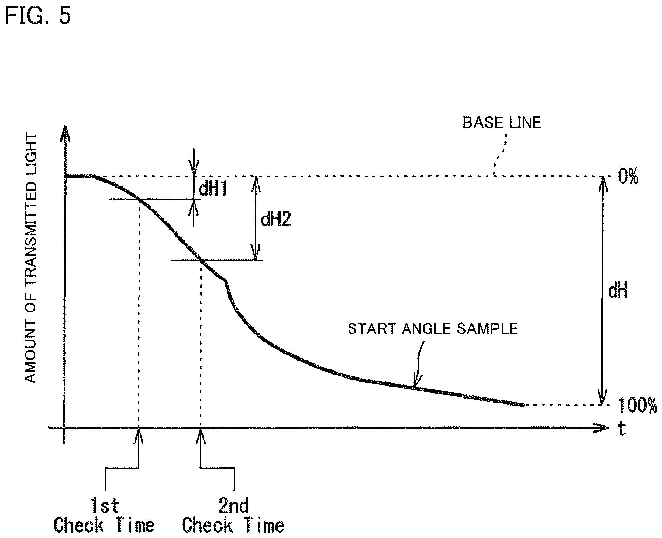

For example, as illustrated in Fig. 5, the early time range (20 seconds for APTT) in the coagulation curve sets a first time point (1st check time [sec]) and a second time point (2nd check time [sec]), and defines a check region between the 1st check time and the 2nd check time. The first time checkpoint is at 4 seconds after the start time of the coagulation curve, for example, and the second time checkpoint is at 8 seconds after the start time of the coagulation curve, for example. Next, the change rate of transmitted light $dH = dH1 - dH2$ within the check region is calculated. The change rate of transmitted light of an abnormal sample is large compared to the change rate of transmitted light of a normal sample. Thus, a threshold value Delta [level] is set. If the difference between $dH1$ and $dH2$ satisfies $dH2 - dH1 \geq$ Delta [level], the ERE flag is set.

In addition, a threshold value dHLimit [level] is set for the change rate of transmitted light $dH$ from the base line to the coagulation reaction end point. If $dH2 - dH1 \geq$ Delta [level] and $dH \leq$ dHLimit (start angle 1), the situation is determined to be a measurement error where the early reaction takes place and an optical change rate attributed to the fibrin formation is not sufficiently large. As a result, the coagulation time is not displayed but the ERE flag is displayed. If $dH2 - dH1 \geq$ Delta [level] and $dH >$ dHLimit (start angle 2), it is judged that the early reaction takes place but the optical change rate is sufficiently large. As a result, an error flag is displayed to indicate a measurement error and then the coagulation time is displayed.

[3.1.3 Early Percent Check]

For a normal blood sample, it takes relatively a long time to cause an optical change attributed to fibrin formation. For a blood sample which causes a gradual optical change, on the other hand, an optical change is caused immediately or relatively soon after a reagent is added to plasma. Thus, it is possible to detect an error by comparing a predetermined threshold value with time until which a checkpoint set at the position of a particular optical detection value is reached.

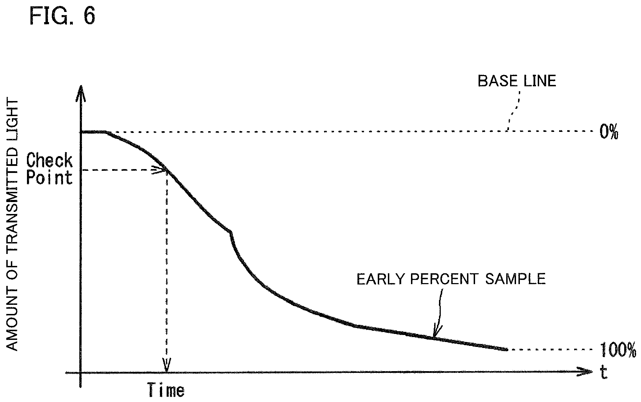

As illustrated in Fig. 6, for example, a Check Point [%] is set in advance for a particular amount of transmitted light in Fig. 6. This check item is intended to monitor whether or not the time at which the amount of transmitted light starts changing is too early. The Check Point is preferably set at the position where the amount of transmitted light starts changing. Next, the time (Time) taken to change to the Check Point [%] is calculated. The Check Point is at the position of 6% considering that the base line is at 0%, for example. The time taken for the amount of transmitted light of an abnormal sample to change to a predetermined amount is shorter compared to the time taken for the amount of transmitted light of a normal sample to change to the predetermined amount. Thus, if a threshold value Limit [sec] is set in advance and Time < Limit is satisfied, it is determined that the time at which the change starts is too early. As a result, the ERE flag is set and displayed on the display 24. The threshold value Limit is 16.8 [sec], for example.

[3.2 Determination of Conformity to Reference Coagulation Curve (Second Check Process)]

If at least one of the early reaction characteristics is detected at step S24, the processing unit 21 determines, as the second check process, the conformity between the coagulation curve and the reference coagulation curve at step S27. In the embodiment, the processing unit 21 executes the second check process if the ERE flag is set as a result of the first check process, but does not execute the second check process if the ERE flag is not set in the first check process. Since the processing load of the second check process is high, it is possible to reduce the processing load by choosing not to execute the second check process if the ERE flag is not set.

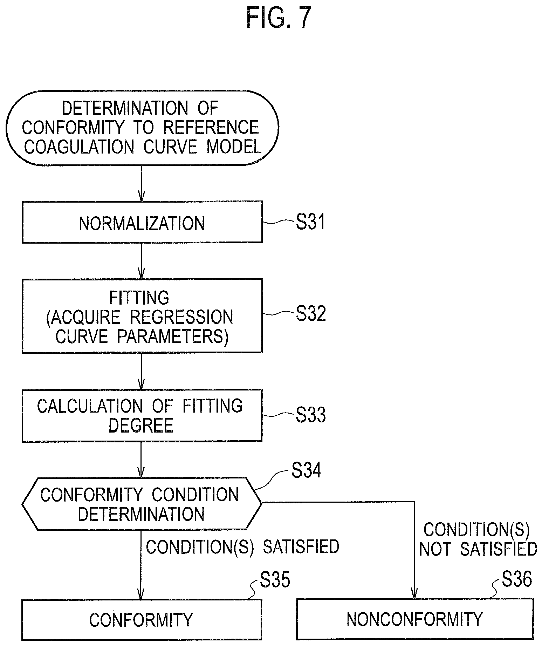

As illustrated in Fig. 7, the conformity determination includes normalization of the coagulation curve data indicated at step S31. At step S32, the normalized coagulation curve data is fitted to the model of the reference coagulation curve (hereinafter simply referred to as a "model"). The model is represented by a function which allows drawing of a curve along the shape of the general coagulation curve.

The general coagulation curve has a shape as illustrated in Fig. 3. Regarding the coagulation time, a reference model is preferably along the shape of the general coagulation curve because the coagulation curve is assumed to have the shape as illustrated in Fig. 3. Since the general coagulation curve is along the sigmoid curve, for example, the model is preferably represented by a sigmoid function. The model may be represented by other functions such as a Gompertz function.

The model represented by the sigmoid curve is given by the following formula:

## [Formula 1]

$$f(x) = \frac{-c_0 - c_1 \cdot x}{1 + e^{-a(x-b)}}$$

In the above model, x is time, and f(x) is the amount of transmitted light normalized at time x. The above model has four parameters $c_0$, $c_1$, a, and b. The processing unit 21 curve-fits the normalized coagulation curve data to the model to perform regression along the reference coagulation curve, thereby calculating the values of the parameters $c_0$, $c_1$, a, and b. In the embodiment, the coagulation curve data which is curve-fitted to the model is not all data during the detection period, but data used to calculate the blood coagulation time. In the embodiment, since the data used to calculate the blood coagulation time is data left after removing data during the mask time from all data during the detection period, the coagulation curve data which is curve-fitted to the model is also data left after removing data during the mask time from all data during the detection period. The coagulation curve data used for the second check process preferably includes data on the optical detection value during the period at least from the coagulation reaction start point to the coagulation reaction end point. The coagulation curve data used for the second check process more preferably includes data before the coagulation reaction start point or data after the coagulation reaction end.

The model which has the calculated values of the parameters $c_0$, $c_1$, a, and b draws the regression curve of the coagulation curve. In the embodiment, the calculating of the parameters $c_0$, $c_1$, a, and b by fitting is equivalent to the obtaining of the regression curve.

It is possible to perform operations for fitting by use of Solver for fitting. As Solver, for example, Nelder-Mead Solver can be used provided by Microsoft Solver Foundation.

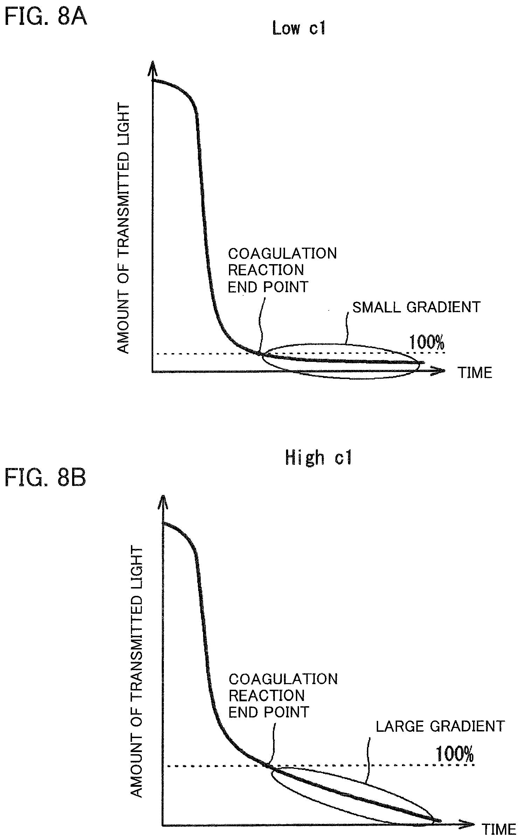

In the model, the parameter $c_0$ represents the magnification factor of the regression curve. The parameter $c_1$ is an indicator for the characteristic of the regression curve after the coagulation reaction end. If the value of $c_1$ is low, the regression curve after the coagulation reaction end has a gradual slope and is almost flat, as illustrated in Fig. 8A. If the value of $c_1$ is high, the amount of transmitted light decreases with time even after the coagulation reaction end and the slope is thus steep, as illustrated in Fig. 8B. If the coagulation curve data includes data after the coagulation reaction end, evaluation of the parameter $c_1$ makes it possible to evaluate the gradient of the portion of the regression curve corresponding to that after the coagulation reaction end.

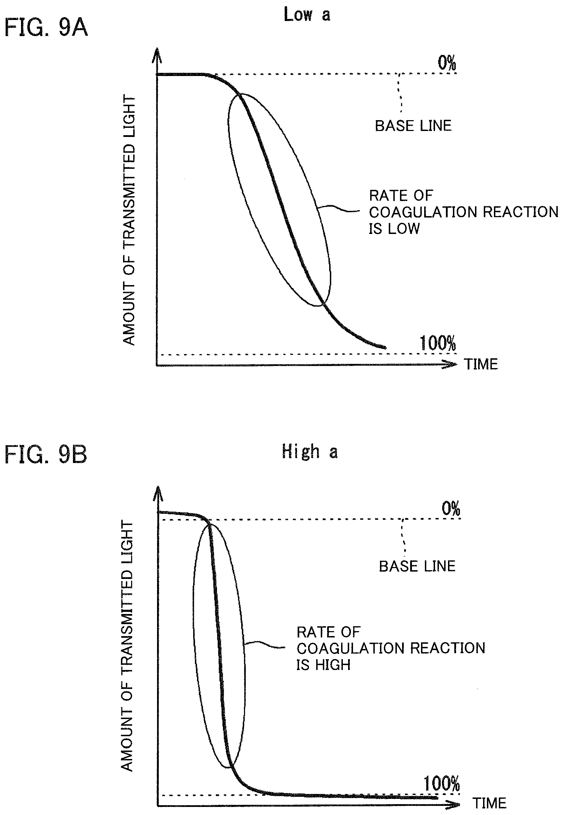

The parameter a is an indicator for the gradient of the regression curve while the coagulation reaction is in progress. If the value of a is low, the coagulation reaction proceeds at a low rate, and the gradient of the regression curve is relatively gradual, as illustrated in Fig. 9A. If the value of a is high, the coagulation reaction proceeds at a high rate, and the regression curve while the coagulation reaction is in progress is steep, as illustrated in Fig. 9B. Evaluation of the parameter a makes it possible to evaluate the gradient of the portion of the regression curve corresponding to that while the coagulation reaction is in progress.

The parameter b represents the inflection point of the regression curve. The inflection point b corresponds to the coagulation time calculated from the regression curve by the percent detection method with the regression curve regarded as the coagulation curve. Evaluation of the difference between the parameter b and the coagulation time calculated from the coagulation curve by the percent detection method makes it possible to evaluate the difference between the coagulation time calculated from the regression curve and the coagulation time calculated from the coagulation curve by the percent detection method.

If the regression curve is obtained, the degree to which the regression curve matches the coagulation curve is calculated at step S33. In the embodiment, the degree to which the regression curve matches the coagulation curve is calculated as the following fitting degree. For the calculation of the fitting degree, the difference $dH = OD_{max} - OD_{min}$ is first calculated between the maximum amount of transmitted light $OD_{max}$ and the minimum amount of transmitted light $OD_{min}$ of the coagulation curve. Next, the error $Error_{(i)} = OD_{(i)} - \{OD_{max} - dH*f(x_{(i)})\}$ is calculated between the value of the regression curve $f(x_{(i)})$ at time i of the regression curve and the amount of transmitted light $OD_{(i)}$ of the coagulation curve at time i of the coagulation curve.

The threshold value Threshold for fitting is set to 2% of the dH (Threshold = 0.02*dH), for example. Operation is performed on the number of data points (number of error data points) at which the error $Error_{(i)}$ is less than the threshold value Threshold, and the fitting degree is calculated as follows:

fitting degree = number of error data points/total number of data points.

The degree to which the regression curve matches the coagulation curve is not limited to the above represented by the fitting degree. The degree may be represented by the total sum of the errors $Error_{(i)}$, for example.

Once the fitting degree is obtained, a conformity condition determination is performed at step S34. The conformity condition determination is determination as to whether or not the ERE flag can be removed. In the embodiment, the conformity condition determination includes the following four conditions:

Condition 1: The fitting degree is 90% (predetermined value) or more.
Condition 2: The value of the parameter a is 0.05 (predetermined value) or more.
Condition 3: The difference between the measured coagulation time and the parameter b (coagulation time obtained from the regression curve) is one second or less.
Condition 4: The parameter $c_1$ is 0.0005 (predetermined value) or less.

Condition 1 is provided in order to guarantee that the regression curve causes the coagulation curve to appropriately regress. Conditions 2 to 4 are provided in order to ensure that the regression curve conforms to the general coagulation curve. A function representing the model can take a shape other than the shape of the general coagulation curve. Thus, it is possible to check whether or not the regression curve conforms to the general coagulation curve by checking whether or not Conditions 2 to 4 are satisfied. In the embodiment, a sigmoid curve satisfying all of Conditions 2 to 4 is the reference coagulation curve. In the embodiment, Conditions 2 to 4 are performed by evaluating the parameters a, b, and $c_1$.

Condition 2 is provided in order to check that the coagulation reaction proceeds at a sufficiently high rate. Condition 3 is provided because a small difference between the measured coagulation time and the parameter b (coagulation time obtained from the regression curve) is important from the viewpoint of conformity. Condition 4 is provided in order to check the curve shape after the reaction end is almost flat.

In the embodiment, the coagulation curve is judged to conform to the reference coagulation curve if all of the four conditions described above are satisfied (step S35), and the coagulation curve is judged not to conform to the reference coagulation curve if one or more of the conditions are not satisfied (step S36). In the embodiment, although the conformity determination between the coagulation curve and the reference coagulation curve is made based on the four conditions described above, the conformity determination may be made based on other conditions. The conformity determination may be made using one or some of the four conditions described above without using all of the four conditions, or may be made after adding a condition to the four conditions described above.

As illustrated in Fig. 1, the ERE flag is removed and the coagulation curve is judged to have no early reaction error if the coagulation curve conforms to the reference coagulation curve. In the case of nonconformity, the ERE flag set in the first check process is retained.

[4. Blood Coagulation Time Output Process]

Once the ERE flag is removed, the coagulation time calculated at step S23 is displayed on the display 24 without the display of an error flag at step S29. If the ERE flag is set, the error flag is displayed. In the case of displaying the error flag, only the error flag may be displayed without displaying the coagulation time, or the error flag may be displayed together with the coagulation time. In such a manner, it is possible to change the output mode for the coagulation time depending on the ERE flag.

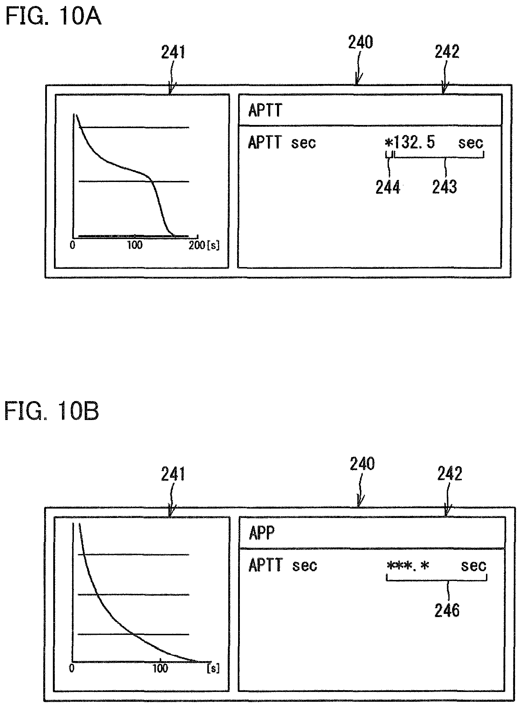

Fig. 10A illustrates an example of an output screen 240 for the coagulation time. This output screen 240 is displayed on the display 24. In the embodiment, the screen 240 includes an output area 2 for the coagulation curve and an output area 242 for the parameters such as the coagulation time. Displayed in the output area 242 is coagulation time (APTT) 243 which is calculated from the coagulation curve displayed in the output area 241. If the ERE flag is set, a symbol for an error flag 244 is added to the display of the coagulation time 243. In Fig. 10A, the error flag 244 is denoted by "*." This error flag 244 indicates that the reliability of the coagulation time 243 being displayed is low, for example.

Fig. 10B illustrates another example of the output screen 240. In Fig. 10B, if the ERE flag is set, the marks "*" denoting the error flag mask the display of the coagulation time (APTT) 246 in the output area 242 so that a user cannot visually recognize the calculated coagulation time. The masking of the coagulation time is useful when, for example, the reliability of the calculated coagulation time is very low.

[5. Conformity Determination Example]

[5.1 Example Where ERE Flag Is Removed]

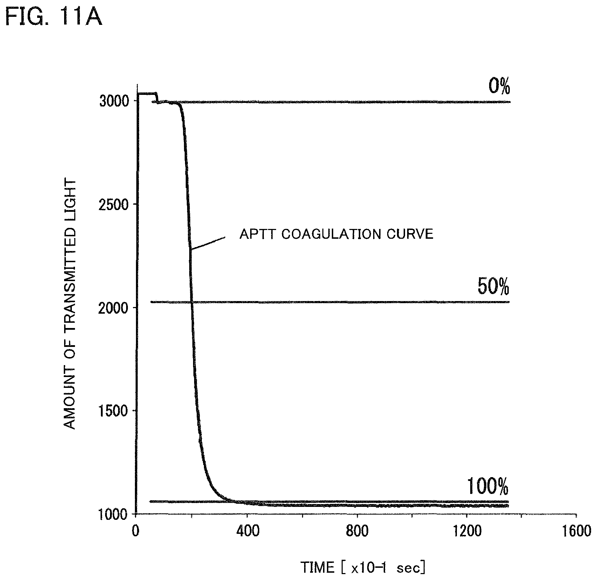

Fig. 11A illustrates an example of an APTT coagulation curve with the ERE flag removed by the conformity determination. Regarding this APTT coagulation curve, the ERE flag is set by an early percent check. The parameters $c_1$, a, and b, and the fitting degree obtained by fitting this APTT coagulation curve to the model are as follows:

$c_1$: 0.000182
a: 0.623
b: 19.68 (the coagulation time obtained from the coagulation curve by the percent detection method is 19.4 seconds)

fitting degree: 91%.

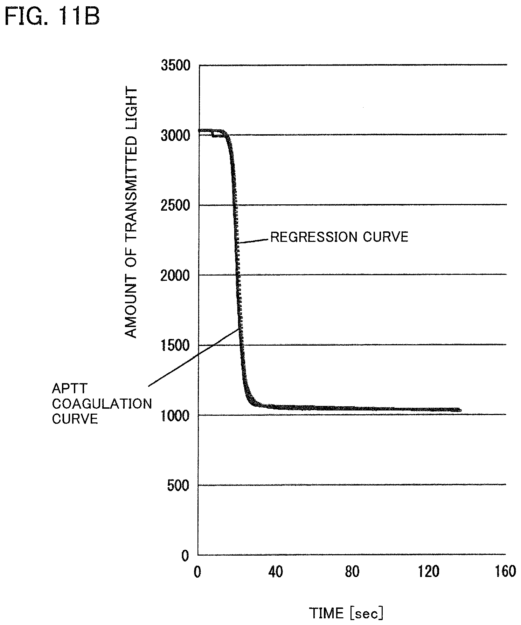

The fitting degree of 91% satisfies Condition 1, a = 0.623 satisfies Condition 2, b = 19.68 satisfies Condition 3, and $c_1$ = 0.000182 satisfies Condition 4. Hence, the ERE flag for the APTT coagulation curve illustrated in Fig. 11 is removed, and the coagulation time of 19.4 seconds is outputted without the error flag. Note that Fig. 11B illustrates the APTT coagulation curve in Fig. 11A and a regression curve obtained by fitting.

[5.2 First Example Where ERE Flag Is Retained]

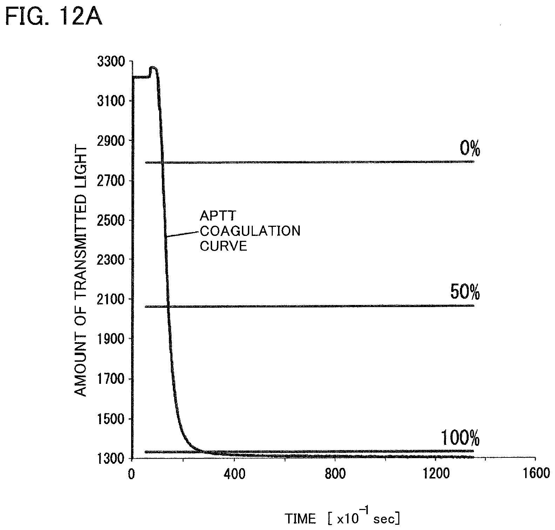

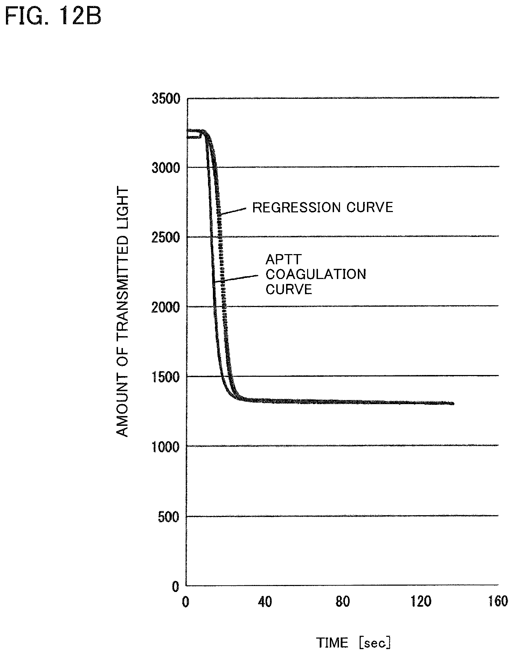

Fig. 12A illustrates a first example of the APTT coagulation curve which is determined as nonconformity by the conformity determination and which has the retained ERE flag. Regarding this APTT coagulation curve, the ERE flag is set by an early percent check. The value of the parameter b obtained by fitting this APTT coagulation curve to the model is 16.98. The coagulation time obtained from the coagulation curve by the percent detection method is 13.7 seconds, which differs from b by 1 second or more and does not satisfy Condition 3. Thus, the determination result is nonconformity, and the ERE flag is retained. Note that Fig. 12B illustrates the APTT coagulation curve in Fig. 12A and a regression curve obtained by fitting.

[5.3 Second Example Where ERE Flag Is Retained]

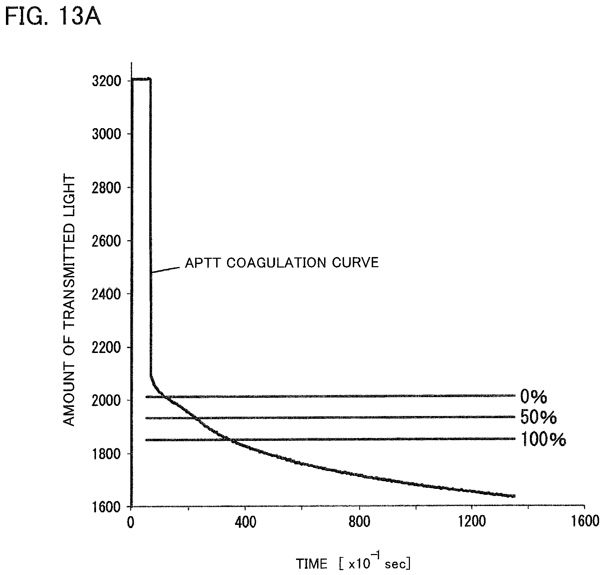

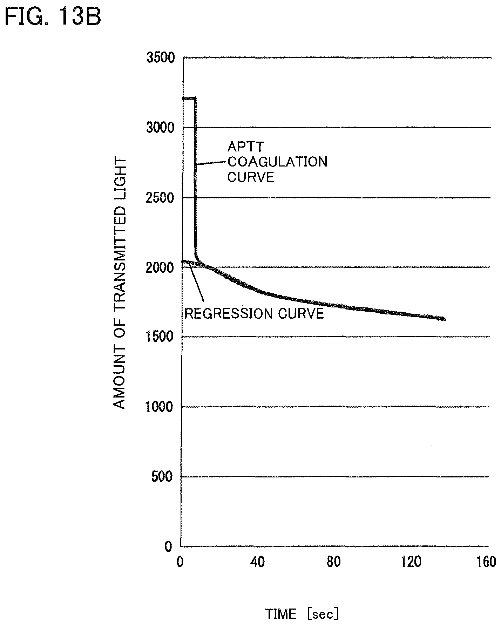

Fig. 13A illustrates a second example of the APTT coagulation curve which is determined as nonconformity by the conformity determination and which has the retained ERE flag. Regarding this APTT coagulation curve, the ERE flag is set by an early percent check. The value of the parameter $c_1$ obtained by fitting this APTT coagulation curve to the model is 0.00406. This value of $c_1$ does not satisfy Condition 4 where $c_1$ is 0.0005 or less. Thus, the determination result is nonconformity, and the ERE flag is retained. Note that Fig. 13B illustrates the APTT coagulation curve in Fig. 13A and a regression curve obtained by fitting.

[5.4 Third Example Where ERE Flag Is Retained]

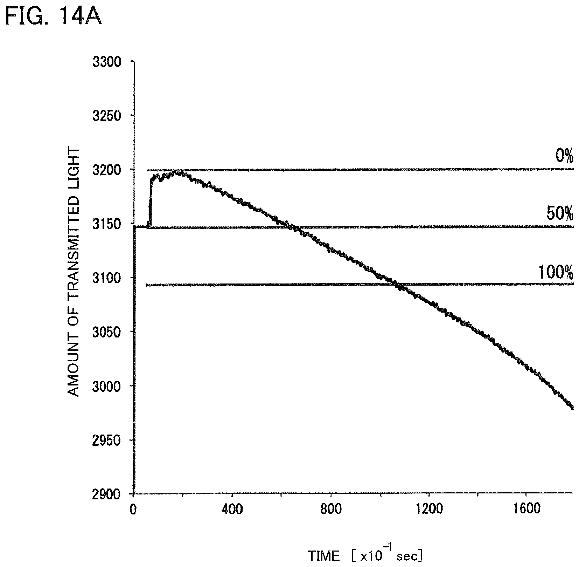

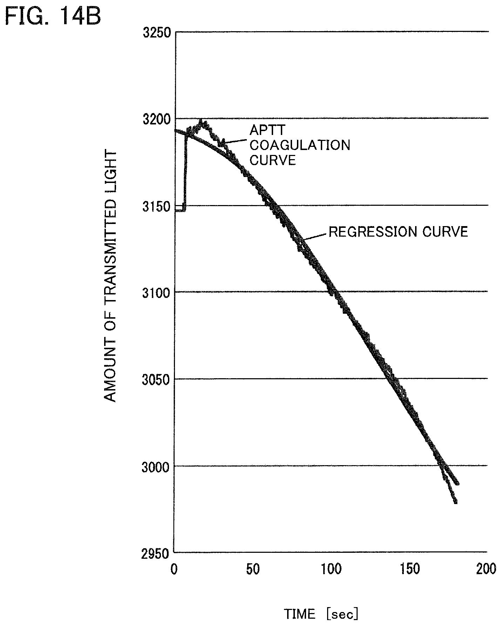

Fig. 14A illustrates a third example of the APTT coagulation curve which is determined as nonconformity by the conformity determination and which has the retained ERE flag. Regarding this APTT coagulation curve, the ERE flag is set by an early percent check. The value of the parameter a obtained by fitting this APTT coagulation curve to the model is 0.024, and the fitting degree is 70%. This value of a does not satisfy Condition 2 where the value of a is 0.05 or more, and Condition 1 is not satisfied where the fitting degree is 90% or more. Thus, the determination result is nonconformity, and the ERE flag is retained. Note that Fig. 14B illustrates the APTT coagulation curve in Fig. 14A and a regression curve obtained by fitting.

[5.5 Comparison Between Example Where ERE Flag Is removed and Example Where ERE Flag Is Retained]

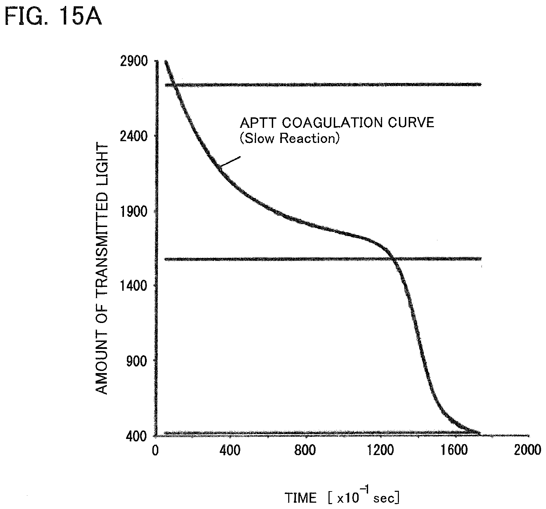

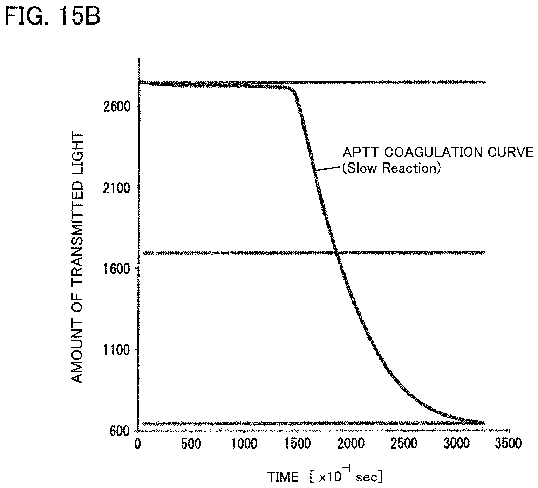

Each of Fig. 15A and Fig. 15B is an example of the APTT coagulation curve for which the ERE flag is set because the reaction progress rate is determined as slow by the slow reaction check. Since the APTT coagulation curve of Fig. 15A is greatly different in shape from a sigmoid curve, the fitting degree is low. Thus, the conformity determination result is nonconformity and the ERE flag is retained. On the other hand, the APTT coagulation curve of Fig. 15B has a high fitting degree for the sigmoid curve even though the reaction is slow, and the gradient of the curve while the reaction is in progress is large. Thus, the calculated coagulation time is reliable. Hence, the conformity determination for the APTT coagulation curve of Fig. 15B results in conformity, and the ERE flag is removed.

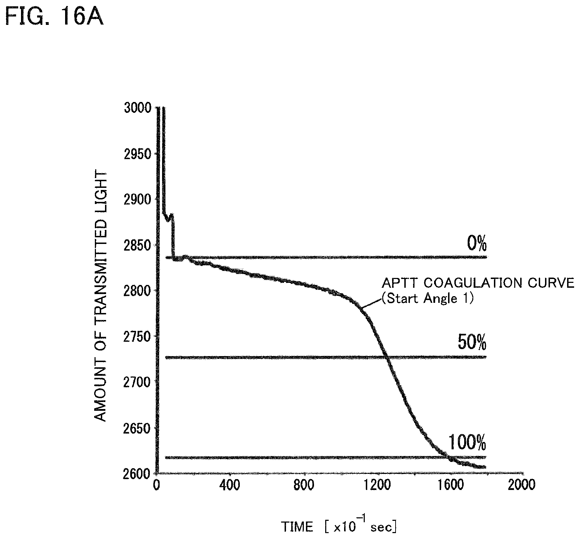

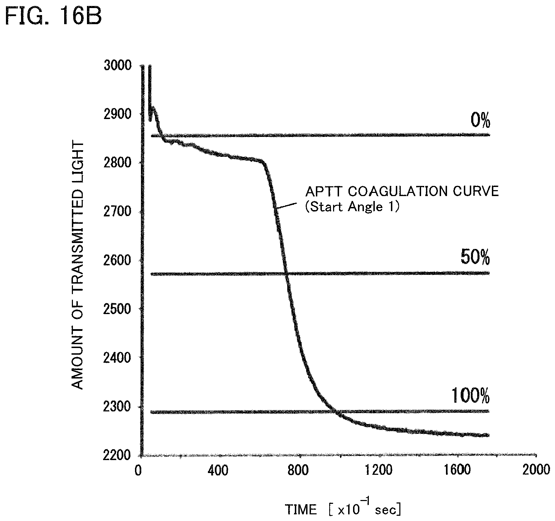

Each of Fig. 16A and Fig. 16B is an example of the APTT coagulation curve for which the ERE flag is set because the gradient within the early section before the start of reaction is determined to be large by the start angle 1 check. The APTT coagulation curve of Fig. 16A is determined as nonconformity by the conformity determination and the ERE flag is retained, whereas the APTT coagulation curve of Fig. 16B is determined as conformity by the conformity determination and the ERE flag is removed.

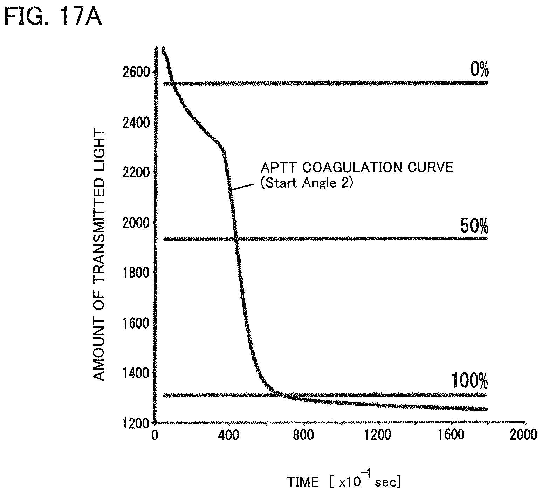

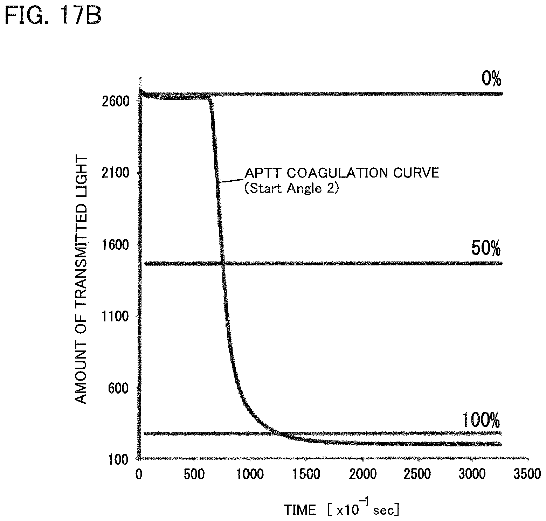

Each of Fig. 17A and Fig. 17B is an example of the APTT coagulation curve for which the ERE flag is set because the gradient within the early section before the start of reaction is determined to be large by the start angle 2 check. The APTT coagulation curve of Fig. 17A is determined as nonconformity by the conformity determination and the ERE flag is retained, whereas the APTT coagulation curve of Fig. 17B is determined as conformity by the conformity determination and the ERE flag is removed.

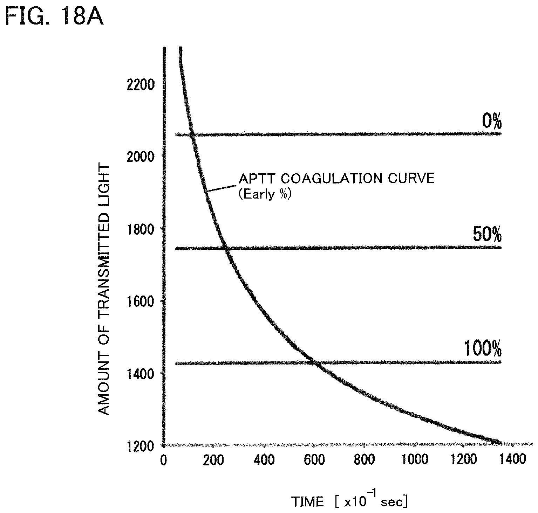

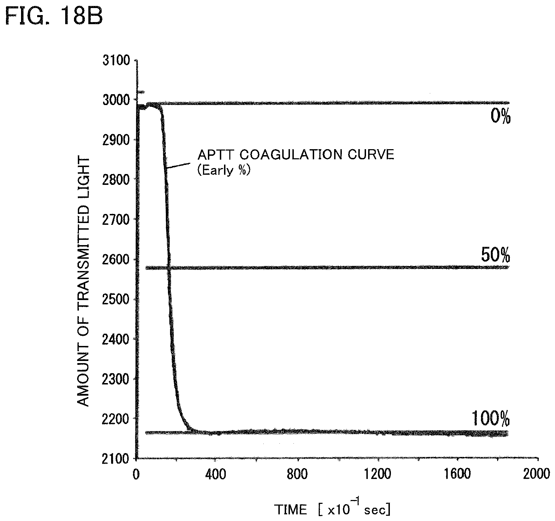

Each of Fig. 18A and Fig. 18B is an example of the APTT coagulation curve for which the ERE flag is set by the early percent check. The APTT coagulation curve of Fig. 18A is determined as nonconformity by the conformity determination and the ERE flag is retained, whereas the APTT coagulation curve of Fig. 18B is determined as conformity by the conformity determination and the ERE flag is removed. The APTT coagulation curve of Fig. 18A shows a decrease in the amount of transmitted light attributed to the early reaction and is greatly different in shape from a sigmoid curve. Thus, the conformity determination result is nonconformity. On the other hand, since the coagulation reaction starts very early in the APTT coagulation curve of Fig. 18B, the coagulation reaction is considered to be detected as the early reaction in the early percent check. However, the entire APTT coagulation curve of Fig. 18B has no early reaction and conforms to the reference coagulation curve. Thus, the conformity determination result is conformity.

[6. Effects of Removing ERE Flag]

Table 1 shows the effects of removing the ERE flag by conformity determination.

[Table 1]

| Number of Sets of Coagulation Curve Data | Number of ERE Flags by First Check Process | | | Number of ERE Flags by Second Check Process | | | Percent FP Reduction |
|---|---|---|---|---|---|---|---|
| | Total | False Positives | True Error | Total | False Positives | True Error | |
| 70,000 | 460 | 239 | 221 | 327 | 106 | 221 | 56% |

As shown in Table 1, among the 70,000 sets of coagulation curve data, the number of events is 460 where the characteristics of the early reaction are extracted by the first check process and the ERE flag is set. These 460 coagulation curve shapes are visually observed. Out of these, the number of curves which are judged to have a reliable coagulation time (false positives) is 239, and the number of curves which are judged to have an unreliable coagulation time (true errors) is 221.

Among the 460 curves, the number of curves for which the ERE flag is retained even after the conformity determination (second check process) is 327. These 327 coagulation curve shapes are visually observed. Out of these, the number of curves which are judged to have a reliable coagulation time (false positives) is 106, and the number of curves which are judged to have an unreliable coagulation time (true errors) is 221.

Table 1 shows that the conformity determination makes it possible to remove only false positives while retaining true errors. The removal rate of false positives is 56% (= (239 - 106)/239), which is a good result.

**Claims**

1. A blood coagulation analysis method comprising:

   calculating blood coagulation time based on data which represents a coagulation curve indicating a temporal change in an optical detection value of a blood specimen added with a measurement reagent; and
   determining an early reaction error by using a result of determination of conformity between a shape of the coagulation curve represented by the data and a shape of a reference coagulation curve.

2. The blood coagulation analysis method according to claim 1, wherein
   the determination of the conformity includes obtaining a regression curve of the data obtained by regression along the reference coagulation curve.

3. The blood coagulation analysis method according to claim 2, wherein
   the conformity determination further includes calculating a degree to which the data matches the regression curve.

4. The blood coagulation analysis method according to claim 2 or 3, wherein
   the conformity determination further includes evaluating a gradient of a portion of the regression curve that corresponds to data obtained while a coagulation reaction is in progress.

**5.** The blood coagulation analysis method according to any one of claims 2 to 4, wherein
the conformity determination further includes evaluating a difference between the blood coagulation time and a coagulation time which is calculated from the regression curve regarded as a coagulation curve.

**6.** The blood coagulation analysis method according to any one of claims 2 to 5, wherein
the conformity determination further includes evaluating a portion of the regression curve that corresponds to data obtained after a coagulation reaction end.

**7.** The blood coagulation analysis method according to any one of claims 2 to 6, wherein
the conformity determination further includes evaluating at least one of parameters which represent the regression curve.

**8.** The blood coagulation analysis method according to any one of claims 1 to 7, wherein
the reference curve is a sigmoid curve.

**9.** The blood coagulation analysis method according to claim 8, wherein
the sigmoid curve is expressed by the following formula:

[Formula 1]

$$f(x) = \frac{-c_0 - c_1 \cdot x}{1 + e^{-a(x-b)}}$$

where x is time, f(x) is an optical detection value at x, and a, b, $c_0$, and $c_1$ are parameters.

**10.** The blood coagulation analysis method according to any one of claims 1 to 9, wherein
the early reaction error is determined by further using a result of detecting at least one of characteristics representative of the early reaction in the coagulation curve.

**11.** The blood coagulation analysis method according to claim 10, wherein
the at least one of the characteristics includes at least one selected from the group consisting of characteristics in which:

time of a change from a first optical detection value to a second optical detection value in the coagulation curve is longer than a reference period;
a change in optical detection value for a predetermined period in the coagulation curve is greater than a reference value; and
time until which the coagulation curve reaches a third optical detection value is shorter than reference time.

**12.** The blood coagulation analysis method according to claim 10 or 11, wherein
the characteristic detection is performed prior to the conformity determination, and
the conformity determination is performed if the at least one of the characteristics is detected, but is not performed if none of the characteristics is detected.

**13.** The blood coagulation analysis method according to any one of claims 1 to 12, wherein
the blood coagulation time is calculated as time until which the optical detection value reaches a coagulation point in the coagulation curve.

**14.** The blood coagulation analysis method according to claim 13, wherein
the coagulation point is a point at which the optical detection value in the coagulation curve changes from a base optical detection value approximately by a certain ratio to a difference between the base optical detection value and an end optical detection value,
the base optical detection value is an optical detection value before coagulation reaction start in the coagulation curve, and
the end optical detection value is an optical detection value at a coagulation reaction end point in the coagulation curve.

**15.** The blood coagulation analysis method according to any one of claims 1 to 14, wherein

the data at least includes optical detection values during a period from coagulation reaction start to coagulation reaction end.

16. The blood coagulation analysis method according to any one of claims 1 to 15, further comprising displaying an error flag on a display device when the early reaction error is determined.

17. A blood coagulation analyzer comprising a processing unit which executes:

calculating blood coagulation time based on data which represents a coagulation curve indicating a temporal change in an optical detection value of a blood specimen added with a measurement reagent; and determining an early reaction error by using a result of determination of conformity between a shape of the coagulation curve data represented by the data and a shape of a reference coagulation curve.

**FIG. 1**

12 → MEASUREMENT UNIT

21 → PROCESSING UNIT

S11

OPTICALLY DETECT BLOOD SPECIMEN

OUTPUT TIME SERIES DATA (COAGULATION CURVE)

S12

PROCESSES RELATED TO COAGULATION TIME

SEARCH FOR BASE LINE — S21

SEARCH FOR COAGULATION REACTION END — S22

CALCULATE COAGULATION TIME — S23

PROCESSES RELATED TO EARLY REACTION ERROR FIRST CHECK PROCESS

FIRST CHECK PROCESS

S24 — IS EARLY REACTION CHARACTERISTIC DETECTED? — NO → NO ERROR DATA (NO ERE FLAG)

YES

S26 — SET ERE FLAG

SECOND CHECK PROCESS

S27 — DETERMINE CONFORMITY TO REFERENCE COAGULATION CURVE MODEL — NONCONFORMITY (ERE FLAG RETAINED)

CONFORMITY

S28 — REMOVE ERE FLAG

NO ERROR DATA

S29 — OUTPUT

FIG. 2

FIG. 3

BASE LINE SEARCH INTERVAL

COAGULATION
REACTION
START POINT

BASE LINE

AMOUNT OF TRANSMITTED LIGHT

0%

COAGULATION REACTION START LEVEL

COAGULATION CURVE

COAGULATION
POINT

COAGULATION DETECTION PERCENT (ex. 50%)

COAGULATION
REACTION END POINT

COAGULATION REACTION
END SEARCH INTERVAL

100%

0

t

MASK TIME

COAGULATION TIME

FIG. 4

FIG. 5

FIG. 6

# FIG. 7

DETERMINATION OF
CONFORMITY TO REFERENCE
COAGULATION CURVE MODEL

NORMALIZATION — S31

FITTING
(ACQUIRE REGRESSION
CURVE PARAMETERS) — S32

CALCULATION OF FITTING
DEGREE — S33

CONFORMITY CONDITION
DETERMINATION — S34

CONDITION(S) SATISFIED

CONDITION(S)
NOT SATISFIED

CONFORMITY — S35

NONCONFORMITY — S36

FIG. 8A

Low c1

FIG. 8B

High c1

FIG. 9A

Low a

FIG. 9B

High a

## FIG. 10A

241  240  242

APTT

APTT sec        *132.5    sec

244    243

## FIG. 10B

241  240  242

APP

APTT sec        ***.*    sec

246

FIG. 11A

FIG. 11B

# FIG. 12A

FIG. 12B

FIG. 13A

FIG. 13B

FIG. 14A

FIG. 14B

FIG. 15A

APTT COAGULATION CURVE
(Slow Reaction)

AMOUNT OF TRANSMITTED LIGHT

TIME $[\times 10^{-1} \text{sec}]$

## FIG. 15B

APTT COAGULATION CURVE
(Slow Reaction)

*Y-axis: AMOUNT OF TRANSMITTED LIGHT (600, 1100, 1600, 2100, 2600)*
*X-axis: TIME [ $\times 10^{-1}$ sec] (0, 500, 1000, 1500, 2000, 2500, 3000, 3500)*

FIG. 16A

FIG. 16B

## FIG. 17A

FIG. 17B

FIG. 18A

# FIG. 18B

APTT COAGULATION CURVE
(Early %)

0%

50%

100%

AMOUNT OF TRANSMITTED LIGHT

TIME [ $\times 10^{-1}$ sec]

Europäisches
Patentamt

European
Patent Office

Office européen
des brevets

# EUROPEAN SEARCH REPORT

Application Number

EP 17 19 8863

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 316 802 A2 (SYSMEX CORP [JP]; DADE BEHRING MARBURG GMBH [DE]) 4 June 2003 (2003-06-04) * par 0014, 0015, 0020, 0040; fig 7-11. * ----- | 1-17 | INV. G01N33/86 G01N33/49 G01N21/82 |
| X | EP 2 927 694 A1 (HITACHI HIGH TECH CORP [JP]) 7 October 2015 (2015-10-07) * par 0045-0046, 0052-0054, 0060, 0075; cl 1-13. * ----- | 1-8, 10-17 | |
| X | US 2012/253693 A1 (INOMATA NORIKAZU [JP] ET AL) 4 October 2012 (2012-10-04) * par 0021-0023, 0104-0107, 0121. * ----- | 1,17 | |

TECHNICAL FIELDS
SEARCHED     (IPC)

G01N

The present search report has been drawn up for all claims

| Place of search | Date of completion of the search | Examiner |
|---|---|---|
| Munich | 13 December 2017 | Motrescu-Hateley, E |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 17 19 8863

This annex lists the patent family members relating to the patent documents cited in the above-mentioned European search report.
The members are as contained in the European Patent Office EDP file on
The European Patent Office is in no way liable for these particulars which are merely given for the purpose of information.

13-12-2017

| Patent document cited in search report | | | Publication date | Patent family member(s) | | | Publication date |
|---|---|---|---|---|---|---|---|
| EP 1316802 | A2 | | 04-06-2003 | AT | 354804 | T | 15-03-2007 |
| | | | | DE | 60218268 | T2 | 31-10-2007 |
| | | | | EP | 1316802 | A2 | 04-06-2003 |
| | | | | JP | 4334171 | B2 | 30-09-2009 |
| | | | | JP | 2003169700 | A | 17-06-2003 |
| | | | | US | 2003138962 | A1 | 24-07-2003 |
| EP 2927694 | A1 | | 07-10-2015 | CN | 104797940 | A | 22-07-2015 |
| | | | | EP | 2927694 | A1 | 07-10-2015 |
| | | | | JP | 6073117 | B2 | 01-02-2017 |
| | | | | JP | 2014106032 | A | 09-06-2014 |
| | | | | US | 2015316531 | A1 | 05-11-2015 |
| | | | | WO | 2014080751 | A1 | 30-05-2014 |
| US 2012253693 | A1 | | 04-10-2012 | JP | 5898410 | B2 | 06-04-2016 |
| | | | | JP | 2012208099 | A | 25-10-2012 |
| | | | | US | 2012253693 | A1 | 04-10-2012 |

EPO FORM P0459

For more details about this annex : see Official Journal of the European Patent Office, No. 12/82

**REFERENCES CITED IN THE DESCRIPTION**

**Patent documents cited in the description**

- JP 5889480 B